# FASCICULE DE BREVET EUROPEEN

(11) **EP 1 513 878 B1**
(45) Date de publication et mention de la délivrance du brevet: **14.03.2007**
(21) Numéro de dépôt: 03748180.1
(22) Date de dépôt: 19.06.2003
(51) Int. Cl.: C07K 14/775, C07K 16/18, G01N 33/92

(54) **COMPOSITIONS ET METHODES POUR LE DOSAGE DE L'APO B48 ET DE L'APO B100**
ZUSAMMENSETZUNGEN UND VERFAHREN ZUM NACHWEIS VOM APO-B48 UND APO-B100
COMPOSITIONS AND METHODS FOR APO-B48 AND APO-B100 ASSAY

(30) Priorité: 19.06.2002 FR 0207542
(43) Date de publication de la demande: 16.03.2005
(73) Titulaire: Genfit, 59120 Loos (FR)
(72) Inventeur: NAJIB, Jamila, 40000 Marrakech (MA); MAJD, Zouher, F-59320 Ennetieres en Weppes (FR)
(74) Mandataire: Tezier Herman, Béatrice
(86) Numéro de dépôt international: PCT/FR2003/001888
(87) Numéro de publication internationale: WO 2004/000884

(56) Documents cités:
- WO-A-98/56938
- DATABASE CA [en ligne] CHEMICAL ABSTRACTS SERVICE, COLUMBUS, OHIO, US; INNERARITY, THOMAS L. ET AL: "Structural relationship of human apolipoprotein B48 to apolipoprotein B10" retrieved from STN Database accession no. 108:126927 CA XP002239297 & JOURNAL OF CLINICAL INVESTIGATION (1987), 80(6), 1794-8, 1987,
- DATABASE WPI , 1986 Derwent Publications Ltd., London, GB; AN 1986-052892 XP002239298 "Monoclonal antibody has high specificity against human apolipoprotein 48" & JP 61 007299 A (DAIICHI KAGAKU YAHUHIN KK), 13 janvier 1986 (1986-01-13)
- DATABASE WPI , 1985 Derwent Publications Ltd., London, GB; AN 1985-285482 XP002239299 "monoclonal antibody against human apolipoprotein 100 produced by hybridoma created by fusion of mouse spleen and myeloma cells" & JP 60 193926 A (TOKYO-DAIGAKU-CHO), 2 octobre 1985 (1985-10-02)
- P-F CHEN ET AL.: "Primary sequence mapping of human apolipoprotein B-100 epitopes" EUROPEAN JOURNAL OF BIOCHEMISTRY, vol. 175, no. 7, juillet 1988 (1988-07), pages 111-118, XP001053294 BERLIN, DE ISSN: 0014-2956

### LISTE DE SEQUENCES

<110> GENFIT SA
<120> Compositions et Méthodes pour le dosage de l'Apo B48 et de l'Apo B100
<130> B0120WO
<140>
   <141>
<160> 2
<170> PatentIn ver. 2.1
<210> 1
   <211> 111
   <212> PRT
   <213> séquence artificielle
<220>
   <223> Description de la séquence artificielle: Peptide synthétique Apo B100
<400> 1
   Lys Ala Ala Ser Gly Thr Thr Gly Thr Tyr Gln Glu Trp Lys Asp Lys
    1               5                  10                  15
   Ala Gln Asn Leu Tyr Gln Glu Leu Leu Thr Gln Glu Gly Gln Ala Ser
               20                  25                  30
   Phe Gln Gly Leu Lys Asp Asn Val Phe Asp Gly Leu Val Arg Val Thr
           35                  40                  45
   Gln Lys Phe His Met Lys Val Lys His Leu Ile Asp Ser Leu Ile Asp
       50                  55                  60
   Phe Leu Asn Phe Pro Arg Phe Gln Phe Pro Gly Lys Pro Gly Ile Tyr
    65                  70                  75                      80
   Thr Arg Glu Glu Leu Ser Thr Met Phe Ile Arg Glu Val Gly Thr Val
                   85                  90                  95
   Leu Ser Gln Val Tyr Ser Lys Val His Asn Gly Ser Glu Ile Leu
               100                 105                 110
<210> 2
   <211> 108
   <212> PRT
   <213> séquence artificielle
<220>
   <223> Description de la séquence artificielle: Peptide synthétique Apo B100
<400> 2
   Ala Phe Thr Asp Leu His Leu Arg Tyr Gln Lys Asp Lys Lys Gly Ile
    1               5                  10                  15
   Ser Thr Ser Ala Ala Ser Pro Ala Val Gly Thr Val Gly Met Asp Met
               20                  25                  30
   Asp Glu Asp Asp Asp Phe Ser Lys Trp Asn Phe Tyr Tyr Ser Pro Gln
           35                  40                  45
   Ser Ser Pro Asp Lys Lys Leu Thr Ile Phe Lys Thr Glu Leu Arg Val
                   50                  55                  60
   Arg Glu Ser Asp Glu Glu Thr Gln Ile Lys Val Asn Trp Glu Glu Glu
    65                 70                  75                  80
   Ala Ala Ser Gly Leu Leu Thr Ser Leu Lys Asp Asn Val Pro Lys Ala
                   85                  90                  95
   Thr Gly Val Leu Tyr Asp Tyr Val Asn Lys Tyr His
               100                 105

## Description

La présente invention concerne des compositions et méthodes pour le dosage ou la détection de l'apolipoprotéine B48 et de l'apolipoprotéine B100 dans des échantillons. Elle concerne notamment une méthode permettant de détecter et de quantifier de façon différentielle l'apolipoprotéine B48 (« Apo B48 ») et l'apolipoprotéine B100 (« Apo B100 »). Cette invention concerne également des produits synthétiques de l'Apo B100, les anticorps correspondants, les kits les comprenant, et leurs utilisations pour détecter, quantifier et/ou suivre dans le temps les quantités d'Apo B48 et/ou d'Apo B100 dans un échantillon biologique. Les produits, matériels et kits décrits ci-dessus peuvent également être utilisés pour moduler de façon différentielle les niveaux d'Apo B48 et/ou d'Apo B100 ou leur activité, *in vitro* ou *in vivo,* et pour réguler le métabolisme lipidique chez un sujet.

Les quantités élevées de triglycérides plasmatiques et la persistance des chylomicrons du régime alimentaire sont des facteurs qui augmentent les risques d'apparition des maladies cardiovasculaires responsables actuellement de la majorité des décès dans le monde (Hokanson and Austin 1996). La quantité élevée de lipides plasmatiques est corrélée au niveau de lipoprotéines Apo B considérées comme étant athérogènes.

Les apolipoprotéines B sont hétérogènes. Les lipoprotéines de très basse densité (VLDL : Very Low Density Lipoprotéin) et les lipoprotéines de basse densité (LDL : Low Density Lipoprotéin) contiennent une isoforme possédant un poids moléculaire apparent d'environ 549 000 Da, laquelle est désignée par l'abréviation B100 dans un système de nomenclature centile, tandis que l'isoforme majoritairement présente dans les chylomicrons (les lipoprotéines postprandiales majoritaires) possède un poids moléculaire apparent de 246 000 Da et est identifiée par l'abréviation B48 dans la même nomenclature. L'Apo B48 est une forme tronquée de l'Apo B100 et est produite par modification post-transcriptionnelle de l'ARN messager codant pour l'Apo B100. Cette modification transforme le codon 2153 (CAA, codant pour une Glutamine) en un codon stop (Davidson, Anant et al. 1995).

L'Apo B48 et l'Apo B100 sont des protéines clés dans le métabolisme des lipoprotéines chez les mammifères. L'Apo B100 est essentielle à l'assemblage des VLDL (Havel 1995) dans le foie humain (Kane 1983) et intervient également dans la capture cellulaire et le catabolisme des LDL. Son intervention a été démontrée dans la voie impliquant le récepteur des LDL (Goldstein and Brown 1977). L'Apo B48 est requise pour l'assemblage des chylomicrons dans l'intestin (Young 1990) (Kane 1983) et est caractéristique des particules "remnant" (ie. résiduelles) de chylomicrons, lesquelles sont considérées comme des facteurs de risque supplémentaires de l'apparition des maladies coronaires (Simons, Dwyer et al. 1987).

Actuellement, on dispose de très peu d'information sur le caractère athérogène des particules de chylomicrons contenant de l'Apo B48 dérivées de l'intestin et des particules contenant de l'Apo B100 dérivées du foie. Il s'avère donc particulièrement pertinent de développer une méthode permettant de détecter et/ou de quantifier de façon différentielle l'Apo B48 et l'Apo B100 de manière à pouvoir, notamment, étudier leurs implications respectives et à mettre en évidence des dérégulations ou dysfonctionnement chez des sujets.

Pour différentes raisons, la mesure de la concentration plasmatique différentielle d'Apo B48 et d'Apo B100 est assez difficile à réaliser. En premier lieu, la séquence en acides aminés de l'Apo B48 est identique à la région N-terminale de l'Apo B100 (représentant 48% de l'Apo B100). En second lieu, l'Apo B48 est présente à très faible concentration dans le plasma, au contraire de l'Apo B100. En troisième lieu, l'expression des épitopes d'Apo B varie en fonction du contenu lipidique des particules.

Des procédés basés sur des méthodes immunologiques et permettant de quantifier l'Apo B48 ont été décrits dans l'art antérieur. En particulier, le procédé d'Uchida et al. (Uchida, Kurano et al. 1998) applique un test ELISA (Enzyme-Linked Immuno-Sorbent Assay) pour quantifier l'Apo B48. La précision et le débit obtenus avec ce procédé sont cependant assez faibles d'où la nécessité de procéder à une purification des anticorps et à leur marquage. De plus, l'anticorps monoclonal utilisé est dirigé contre la région C-terminale de l'Apo B48 humaine. Cet anticorps est obtenu grâce à la conformation différentielle de l'Apo B48 dans les particules contenant de l'Apo B100, ce qui peut conduire à une spécificité et à une affinité différentes des anticorps en fonction de la composition lipidique des lipoparticules.

D'autres documents décrivent une quantification de l'Apo B48 à l'aide de techniques non immunochimiques telles que l'électrophorèse capillaire (Proctor and Mamo 1997), la chromatographie liquide réalisée sur un système HPLC (Hidaka, Kojima et al. 1990) (Wagner, Nustede et al. 1987) ou le SDS PAGE (Karpe and Hamsten 1994) (Smith, Proctor et al. 1997). Ces méthodes présentent différents inconvénients : elles possèdent un faible débit et nécessitent des étapes de préparation des lipoprotéines et de délipidation pour améliorer la précision des résultats. Ces techniques sont par ailleurs beaucoup moins sensibles que les méthodes immunochimiques.

### Résumé de l'Invention

La présente invention fournit une nouvelle stratégie pour produire des peptides synthétiques spécifiques de l'Apo B100 et de nouvelles méthodes permettant de détecter et de quantifier de façon différentielle l'Apo B100 et l'Apo B48. La quantification différentielle est typiquement réalisée par dosage de l'Apo B totale (Apo B100 et Apo B48) dans un échantillon, de préférence à l'aide d'un anticorps polyclonal anti-Apo B. L'échantillon est ensuite débarrassé de l'Apo B100 à l'aide d'un anticorps anti-Apo B100 spécifique, de manière à quantifier directement et spécifiquement l'Apo B48.

La présente invention décrit plus particulièrement de nouvelles méthodes de production d'anticorps spécifiques de l'Apo B100 à l'aide de peptides synthétiques spécifiques de l'Apo B100. L'invention décrit également de tels anticorps, des kits les comprenant et leurs utilisations pour détecter, quantifier, purifier et/ou suivre l'évolution des quantités d'Apo B100 et d'Apo B48 dans le sérum ou le plasma. Ces anticorps fournissent également une nouvelle approche pour moduler l'activité de l'Apo B *in vitro* ou *in vivo,* et pour réguler le métabolisme lipidique chez un sujet.

Un premier objet particulier de l'invention concerne un peptide synthétique spécifique de l'Apo B100 substantiellement pur, comprenant la séquence SEQ ID NO : 1 ou la séquence SEQ ID NO : 2, ou un fragment immunogène ou un dérivé de ce peptide.

Un autre objet de l'invention concerne une méthode de production d'anticorps anti-Apo B100 comprenant une étape d'immunisation à l'aide d'un peptide synthétique spécifique de l'Apo B100 tel que défini ci-dessus. Cette invention comprend également les anticorps préparés selon cette méthode, de même que, plus généralement, les anticorps capables de lier un peptide tel que défini ci-dessus ainsi que les fragments ou dérivés de tels anticorps.

Un autre aspect de cette invention concerne une méthode de détection ou de dosage d'Apo B100 et d'Apo B48 dans des échantillons biologiques (notamment dans des lipoparticules préparées ou dans des échantillons de plasma ou de sérum), à l'aide d'un anticorps (incluant un fragment ou un dérivé de ce dernier) tel que défini ci-dessus.

Un autre objet de cette invention concerne une méthode de détection de la présence, d'une prédisposition ou d'un risque de développer un désordre lié au métabolisme des lipides chez un sujet, comprenant la détection *in vitro*, dans un échantillon dudit sujet, de l'Apo B100 et de l'Apo B48 à l'aide d'un anticorps (incluant un fragment ou un dérivé de ce dernier) tel que défini ci-dessus. La méthode est particulièrement adaptée à la détection de la présence, d'une prédisposition ou d'un risque de développement de l'athérosclérose ou d'une pathologie cardiovasculaire telle que par exemple une maladie coronaire.

A cet égard, un objet spécifique de l'invention concerne une méthode de détection et/ou de suivi de la formation, du développement ou de la progression de l'athérosclérose chez un sujet, comprenant la détection *in vitro,* dans un échantillon dudit sujet, de la quantité ou du niveau d'Apo B100 et/ou d'Apo B48 à l'aide d'un anticorps (incluant un fragment ou un dérivé de ce dernier) tel que décrit ci-dessus.

Un autre objet de cette invention concerne une méthode de détection ou de suivi chez un sujet de la capture cellulaire de lipoprotéines riches en triglycérides et de LDL, comprenant la détection *in vitro* de particules contenant de l'Apo B à l'aide d'un anticorps (incluant un fragment ou un dérivé de ce dernier) tel que défini ci-dessus.

Le sujet est généralement un mammifère, de préférence un être humain, de façon encore plus préférée un sujet présentant un risque de développer des maladies cardiovasculaires liées à un désordre du métabolisme des lipides tel qu'une maladie coronaire, ou encore un sujet présentant une telle maladie.

Un autre objet de cette invention concerne une composition pharmaceutique comprenant un anticorps (incluant un fragment ou un dérivé de ce dernier) tel que défini ci-dessus et un excipient ou un véhicule acceptable sur le plan pharmaceutique.

### Description Détaillée de l'Invention

Comme indiqué ci-dessus, un aspect de la présente invention concerne un peptide synthétique spécifique de l'Apo B100 substantiellement pur comprenant la séquence SEQ ID NO : 1 ou la séquence SEQ ID NO : 2, ou un fragment immunogène ou un dérivé de ce peptide. On entend par « peptide synthétique spécifique de l'Apo B100 » tous peptides synthétisés de façon artificielle mimant une partie de la protéine Apo B100 native et comportant des régions ou épitopes propres à cette protéine et essentiellement absents d'autres protéines. Il s'agit notamment de peptides comprenant la séquence d'une région de l'Apo B100 absente dans les autres formes de Apo B, particulièrement dans l'Apo B48. Le terme « substantiellement pur » indique que le peptide est essentiellement dépourvu de séquences d'acides aminés présentes dans l'Apo B48 et/ou de protéines contaminantes normalement présentes ou associées à l'Apo B dans des lipoparticules, telles que notamment l'Apo Al. Le terme « synthétique » indique que le peptide n'est pas une molécule obtenue de façon naturelle mais qu'il a été préparé grâce à un procédé artificiel (e.g., synthèse chimique, assemblage, etc.) notamment tel que décrit dans les exemples. Dans ce contexte, le peptide synthétique spécifique de l'Apo B100 de l'invention est préférentiellement essentiellement non-glycosylé.

La présente invention démontre à présent que des peptides synthétiques spécifiques de l'Apo B100 peuvent être produits et utilisés pour générer des anticorps anti-Apo B100 spécifiques. L'invention démontre par ailleurs que de tels anticorps sont capables de se lier de façon spécifique à l'Apo B100 obtenue soit de façon naturelle soit sous la forme d'un antigène soluble, soit incluse dans des lipoparticules. L'invention démontre également que de tels anticorps peuvent se lier à différentes lipoparticules contenant de l'Apo B100 (IDL (Intermediary Density Lipoprotein), VLDL et LDL), et présentent des propriétés d'immuno-précipitation. Ces peptides synthétiques spécifiques de l'Apo B100 et leurs anticorps correspondants représentent ainsi de nouveaux produits particulièrement avantageux pour détecter l'Apo B100 et quantifier de façon différentielle l'Apo B100 et l'Apo B48.

Plus particulièrement, un peptide synthétique spécifique de l'Apo B100 préféré de l'invention comprend la séquence SEQ ID NO : 1, telle que décrite ci-dessous, ou un fragment immunogène ou un dérivé de ce peptide.

Ce peptide correspond aux résidus 4105 à 4215 de la séquence de l'Apo B humaine mature.

Selon une variante de l'invention, un peptide synthétique spécifique de l'Apo B100 préféré de l'invention comprend la séquence SEQ ID NO : 2, telle que décrite ci-dessous, ou un fragment immunogène ou un dérivé de ce peptide.

Ce peptide correspond aux résidus 3955 à 4062 de la séquence de l'Apo B humaine mature.

Les peptides de l'invention comportent moins de 200 acides aminés, plus préférentiellement moins de 180 acides aminés.

Comme illustré dans les exemples, les peptides de l'invention (comprenant la séquence SEQ ID NO: 1 ou la séquence SEQ ID NO : 2) peuvent être préparés de façon particulièrement avantageuse par synthèse en phase solide, plus particulièrement en utilisant une stratégie Boc/Bzl (Merrifield 1963).

Un objet particulier de l'invention concerne un peptide synthétique spécifique des Apo B caractérisé en ce qu'il comprend des épitopes spécifiques de l'Apo B100 et en ce qu'il est dépourvu d'épitopes spécifiques de l'Apo B48, et en ce qu'il comprend la séquence SEQ ID NO : 1 ou la séquence SEQ ID NO : 2.

Les peptides peuvent être solubles, purifiés ou complexés à une molécule porteuse, telle que KLH (Keyhole Limpet Hemocyanin) ou la sérum albumine par exemple, ou encore toute autre molécule inerte (e.g., synthétique) telle qu'une bille, etc.. Selon un mode particulier de réalisation de l'invention, les peptides sont couplés à une molécule porteuse. Ils le sont en particulier dans le cadre de leur utilisation pour produire des anticorps. Le couplage peut être réalisé selon des méthodes conventionnelles connues de l'homme du métier (Vaitukaitis, Robbins et al. 1971) (Bassiri 1979).

Les peptides peuvent également être conjugués ou couplés à une molécule polypeptidique hétérologue, telle qu'une molécule biologiquement active par exemple. Le caractère hétérologue désigne tout peptide qui n'est pas originaire d'une molécule d'Apo B humaine.

Un objet particulier de l'invention concerne une composition caractérisée en ce qu'elle comprend un peptide synthétique comprenant la séquence SEQ ID NO : 1 ou la séquence SEQ ID NO : 2, et en ce qu'elle est dépourvue d'autre apolipoprotéine.

Les peptides peuvent être utilisés dans le cadre de méthodes de criblage de composés modulant l'activité de l'Apo B, de méthodes de titration, comme contrôles, standards ou pour calibrer des méthodes d'analyse. Ils peuvent également être utilisés pour moduler l'activité de l'Apo B100, avantageusement de manière spécifique. Ils sont également particulièrement utiles pour produire des anticorps anti-Apo B100.

A cet égard, un autre objet de l'invention concerne tout anticorps capable de se lier à un peptide tel que défini ci-dessus, préférentiellement de manière spécifique. De préférence, les anticorps selon l'invention présentent la capacité de précipiter l'Apo B100 (immuno-précipitants). L'anticorps peut être polyclonal ou monoclonal. Avantageusement, lorsque les anticorps sont monoclonaux, ils sont présents sous forme de mélange d'anticorps monoclonaux. De plus, le terme anticorps inclut également tous fragments et dérivés d'anticorps, en particulier les fragments et dérivés desdits anticorps monoclonaux ou polyclonaux présentant substantiellement la même spécificité antigénique. Ces derniers comprennent des fragments d'anticorps (e.g., Fab, F(ab')2, CDRs, etc.), des anticorps humanisés, des anticorps polyfonctionnels, des anticorps monocaténaires (ScFv), etc.. Les anticorps de l'invention peuvent être produits à l'aide de méthodes conventionnelles, comprenant l'immunisation d'un animal et la récupération de son sérum (polyclonal) ou de cellules spléniques (de manière à produire des hybridomes par fusion avec des lignées cellulaires appropriées).

Des méthodes de production d'anticorps polyclonaux à partir d'espèces animales variées incluant les rongeurs (souris, rats, etc.), les primates, les chevaux, les cochons, les moutons, les lapins, la volaille, etc. sont décrites par exemple dans Vaitukaitis et al. (Vaitukaitis, Robbins et al. 1971). L'antigène est combiné avec un adjuvant (e.g., Freud's adjuvant) et administré à un animal, typiquement par injection sous-cutanée. Des injections répétées peuvent être réalisées. Les échantillons sanguins (immun sérum) sont collectés et les immunoglobulines sont séparées.

Des méthodes de production d'anticorps monoclonaux à partir de différentes espèces animales peuvent être trouvées par exemple dans Harlow et al. (Harlow 1988) ou dans Kohler et al. (Kohler and Milstein 1975). Ces méthodes comprennent l'immunisation d'un animal avec un antigène, suivie de la récupération des cellules spléniques qui sont ensuite fusionnées avec des cellules immortalisées, telles que des cellules de myélome. Les hybridomes résultant produisent des anticorps monoclonaux et peuvent être sélectionnés par dilutions limites de manière à isoler les clones individuels. Les anticorps peuvent également être produits par sélection de banques combinatoires d'immunoglobulines, telles que celles divulguées par exemple dans Ward et al. (Ward, Gussow et al. 1989).

Des anticorps préférés de l'invention sont préparés par immunisation d'un animal non-humain à l'aide d'un peptide synthétique spécifique de l'Apo B100 substantiellement pur tel que décrit ci-dessus, comprenant de préférence la séquence SEQ ID NO : 1 ou la séquence SEQ ID NO : 2, ou un fragment immunogène ou un dérivé de ce peptide, e.g., un sous-fragment comprenant au moins un épitope, ou un mélange de fragments immunogènes ou dérivés des SEQ ID NO : 1 et SEQ ID NO : 2 (oligopeptides) et récupération des anticorps ou des cellules productrices d'anticorps.

Les fragments Fab ou F(ab')2 peuvent être produits par digestion à l'aide d'une protéase selon les techniques conventionnelles. Les anticorps humanisés peuvent être préparés selon l'une des méthodes décrites, par exemple, dans Riechmann et al. (Riechmann 1988) (Jones 1986).

L'invention concerne également une méthode de production d'anticorps anti-Apo B100 spécifiques, comprenant l'administration (e.g., l'injection) d'un peptide de séquence SEQ ID NO : 1 ou de séquence SEQ ID NO : 2, ou un fragment immunogène ou un dérivé de ce peptide, tel que décrit ci-dessus, à un animal non-humain et la récupération des anticorps ou des cellules productrices d'anticorps.

La méthode permet avantageusement la production d'anticorps spécifiques et immuno-précipitants. La spécificité peut être vérifiée par la démonstration de l'absence d'une réaction croisée avec d'autres protéines circulantes sanguines comprenant de l'Apo B48. Plus généralement, la spécificité indique que les anticorps sont capables de se lier à l'Apo B100 avec une affinité supérieure à tout autre antigène. Comme illustré dans les exemples, les anticorps polyclonaux de la présente invention sont immuno-précipitants, et peuvent ainsi être utilisés pour détecter l'Apo B100 ou pour doser de façon différentielle l'Apo B100 et l'Apo B48 avec une grande efficacité.

Les anticorps peuvent être couplés à des fragments hétérologues tels que des toxines, des marqueurs, des médicaments ou tout autre agent thérapeutique, de façon covalente ou non, soit directement, soit par l'intermédiaire d'agents de couplage. Les marqueurs peuvent être choisis parmi les radio-marqueurs, des enzymes, des agents fluorescents, des particules magnétiques, etc.. Des toxines préférées sont par exemple la toxine diphtérique, la toxine botulique, etc.. Les médicaments ou les agents thérapeutiques sont choisis notamment parmi des lymphokines, des antibiotiques, des séquences anti-sens, des facteurs de croissance, etc.. Des méthodes permettant de réaliser le couplage des anticorps et de tels fragments hétérologues sont illustrées par exemple dans le brevet US 4,277,149 et dans le brevet US 3,996,345.

Les anticorps de l'invention possèdent de nombreuses applications incluant des applications thérapeutiques, prophylactiques, diagnostiques, de purification, de détection, etc..

*In vitro,* ils peuvent être utilisés comme agents de criblage ou pour purifier des antigènes provenant d'échantillons divers, incluant des échantillons biologiques variés (e.g., des échantillons sanguins). Ils peuvent également être utilisés pour détecter ou quantifier la présence ou la quantité d'Apo B100 ou indirectement d'Apo B48 dans les lipoparticules contenant l'Apo B présentes au sein d'un échantillon collecté à partir d'un sujet, typiquement, un échantillon sanguin provenant d'un mammifère ou, de manière préférée, d'un être humain.

A cet égard, un autre objet de l'invention concerne une méthode de détection ou de quantification de l'Apo B100, au sein d'un échantillon biologique, comprenant la mise en contact de l'échantillon avec un anticorps tel que défini ci-dessus (incluant des fragments ou des dérivés de ce dernier) et la détection de la présence de complexes immuns antigène-anticorps.

La quantification est typiquement réalisée de manière différentielle, c'est-à-dire par dosage de l'Apo B totale (Apo B100 et Apo B48) dans un échantillon, qui est ensuite débarrassé de l'Apo B100 à l'aide d'un anticorps anti-Apo B100 spécifique, permettant de quantifier directement et spécifiquement l'Apo B48. La concentration d'Apo B100 est obtenue par différence entre la mesure totale d'Apo B et celle d'Apo B48. Cette méthode permet donc la détermination des niveaux d'Apo B100 et d'Apo B48 dans un échantillon, par détermination des quantités (relatives) de complexes immuns dans l'échantillon (avant et après la récupération de l'Apo B100) et la comparaison à des conditions standards ou à une courbe de calibrage par exemple.

Une méthode de détection ou de quantification différentielle préférée comprend donc avantageusement les étapes suivantes :
a. détermination de la quantité d'Apo B totale dans un échantillon avec un anticorps anti-Apo B totale,
b. élimination de l'Apo B100 ou des lipoparticules contenant l'Apo B100 dudit échantillon, à l'aide d'anticorps anti-Apo B100 spécifiques tels que décrits ci-dessus, et
c. détermination de la quantité d'Apo B48 dans l'échantillon obtenu à l'issue de l'étape (b) ou dans les lipoparticules ne contenant pas d'Apo B100 au sein de cet échantillon, avec un anticorps anti-Apo B totale.

Par déduction, les quantités obtenues en (a) et (c) permettent la détermination des quantités d'Apo B100 (ou de lipoparticules contenant de l'Apo B100) dans l'échantillon.

Les anticorps utilisés peuvent être polyclonaux, monoclonaux ou des fragments ou dérivés de ceux-ci, seuls, modifiés et/ou en mélange. Ils peuvent en particulier être mis en oeuvre sous forme soluble ou immobilisée à des supports, notamment des billes, plaques, colonnes, etc.

L'anticorps anti-Apo B totale est avantageusement composé d'un mélange de monoclonaux ou d'un polyclonal, afin d'assurer une détection ou une quantification complète de l'antigène, dans ses différentes formes. Par ailleurs, l'anticorps mis en oeuvre dans les étapes a) et c) peut être identique ou différent. Généralement, il s'agit du même anticorps.
On utilise typiquement un anticorps anti-Apo B totale polyclonal. L'anticorps polyclonal anti-Apo B totale peut être obtenu par toute technique connue de l'homme du métier, notamment par immunisation au moyen de l'Apo B entière. De tels anticorps sont décrits notamment dans les publications de Li (Li, Wu et al. 1997) et Levinson (Levinson and Wagner 1993).

Avantageusement, au cours de l'étape b), pour éliminer de manière sélective l'Apo B100 présente dans l'échantillon, on utilise un ou des anticorps spécifiques anti-Apo B100 selon l'invention, dont la fixation sur l'Apo B100 provoque la précipitation de l'ensemble. A titre d'exemples non limitatifs, on peut donc utiliser soit des anticorps polyclonaux, soit un mélange d'anticorps monoclonaux, soit des anticorps (polyclonaux ou monoclonaux) modifiés de manière à provoquer la précipitation des complexes immuns Apo B100-anticorps.

Dans une première variante, les anticorps spécifiques anti-Apo B100 mis en oeuvre dans l'étape b) sont des anticorps polyclonaux, possédant la propriété d'être immuno-précipitants.

Dans une autre variante, on peut également utiliser un mélange d'anticorps monoclonaux anti-Apo B spécifiques ou des anticorps monoclonaux modifiés (par exemple liés avec du latex ou d'autres particules) de manière à provoquer la précipitation des complexes immuns Apo B-anticorps.

Un autre moyen d'éliminer les lipoprotéines contenant l'Apo B100 consiste à fixer des anticorps anti-Apo B100 spécifiques à des protéines A ou G ou à d'autres molécules chimiques (polymères chimiques ou résines tels que le polyuréthane, le sépharose activé, etc.). Ces complexes peuvent être liés à des billes magnétiques, permettant l'élimination de l'Apo B100 par application d'un champs magnétique (e.g., précipitation ou séparation magnétique).

La méthode d'analyse selon l'invention (et plus spécifiquement la détection ou la quantification de complexes immuns antigène-anticorps) peut être réalisée à l'aide de techniques conventionnelles telles que des méthodes ELISA (immuno-essai direct ou compétitif), RIA (Radio Immuno Assay), EIA (Electro immuno assay également nommé Electro immuno diffusion), turbidimétrie, ou toute autre méthode immunologique. Cependant, un objet particulièrement préféré de l'invention concerne une méthode d'analyse néphélométrique. En effet, comme indiqué ci-dessus, les anticorps sont spécifiques et peuvent immuno-précipiter l'Apo B100 dans un échantillon permettant ainsi de doser de façon différentielle l'Apo B48 avec un anticorps polyclonal (ou un mélange d'anticorps monoclonaux ou un anticorps monoclonal modifié de manière à provoquer la précipitation des complexes immuns Apo B-anticorps) anti-Apo B totale.

Dans la méthode d'analyse néphélométrique, l'intensité lumineuse émise par les particules en suspension est mesurée à l'aide d'un analyseur. Les particules sont formées lors de la réaction d'immuno-précipitation qui se produit, dans un milieu tampon stimulant la formation des polymères, quand un anticorps spécifique est mis en contact avec un antigène spécifique. Le complexe comprenant un antigène et un anticorps spécifique de l'antigène se forme à un niveau qui augmente graduellement dans un premier temps, puis augmente rapidement et finalement passe par une valeur pic qui est proportionnelle à la concentration d'antigènes. La méthode d'analyse est basée sur la mesure du taux maximum de changement de l'intensité du signal lumineux qui est corrélé (et peut être converti) à la concentration de l'antigène. Typiquement, la méthode d'analyse néphélométrique est réalisée à l'aide de systèmes immunochimiques Beckman (IMMAGE, Beckman Coulter, Foster city, CA, USA) qui présentent les résultats sur une table alphamétrique. La méthode néphélométrique de l'invention est particulièrement avantageuse dans la mesure où elle est rapide et reproductible et permet d'obtenir un débit élevé. En effet, cette méthode ne nécessite que quelques secondes pour analyser chaque échantillon, les techniques de l'art antérieur imposant, à titre de comparaison, un délai d'un jour. D'autre part la technique selon l'invention peut être facilement automatisée. Par ailleurs, le coefficient de variation pour la détection d'Apo B (quelque soit l'isoforme considérée) est de 4 % seulement dans le cadre de l'invention, contre 10% dans l'art antérieur.

Un objet particulier de l'invention concerne ainsi une méthode de quantification différentielle de l'Apo B48 et de l'Apo B100 dans les lipoparticules présentes dans un échantillon biologique, comprenant la mise en contact de l'échantillon (ou d'une dilution de ce dernier) avec un anticorps tel que décrit ci-dessus (incluant les fragments ou les dérivés de celui-ci). Brièvement, dans un premier temps, un échantillon biologique est dosé par un anticorps anti-Apo B totale dans un système immuno-néphélométrique permettant ainsi la quantification de l'Apo B totale circulante. Dans un deuxième temps l'échantillon biologique est débarrassé de son Apo B100 par mise en contact de cet échantillon avec un anticorps anti-Apo B100 spécifique (généré préférentiellement grâce aux peptides de la SEQ ID NO : 1 ou de la SEQ ID NO : 2). Enfin la quantité d'Apo B48 de l'échantillon débarrassé de son Apo B100 est dosée par l'anticorps anti-Apo B totale de la même façon que l'échantillon brut. La quantité d'Apo B100 peut ainsi être obtenue par simple différence entre la quantité de Apo B totale et la quantité de l'Apo B48. Typiquement les différentes dilutions de l'échantillon biologique et/ou les anticorps anti-Apo B totale et/ou anti-Apo B100 sont soumis à un traitement préalable à la mise en contact avec l'échantillon, en vue de supprimer les protéines non immunoglobulines et/ou de concentrer l'anticorps. Le traitement comprend typiquement la mise en contact des anticorps avec du polyéthylène glycol (PEG), tel que décrit par exemple dans Ritchie et al. (Ritchie 1972). Typiquement, 0,5 à 1 µg d'anticorps spécifiques sont utilisés dans cette méthode d'analyse, bien que l'homme de l'art puisse utiliser différentes quantités sans se démarquer d'une façon significative de la présente invention.

Dans une méthode d'analyse néphélométrique, des anticorps polyclonaux sont généralement utilisés.

La méthode d'analyse peut être réalisée sur différents échantillons biologiques, incluant notamment du sang, du plasma, du sérum, du fluide interstitiel, du liquide céphalorachidien, du surnageant de cultures cellulaires, etc.. L'échantillon peut être collecté chez un sujet (e.g., un sujet humain) et utilisé directement pour mettre en oeuvre la méthode d'analyse. De manière alternative, l'échantillon peut être dilué et/ou stocké (par exemple sous forme congelée) pour être testé plus tard. L'invention permet également la mesure des concentrations d'Apo B48 et d'Apo B100 contenues dans les lipoparticules grâce à l'utilisation des anticorps anti-Apo B totaux et anti-Apo B100 spécifiques, avec une efficacité, une sûreté et un débit élevé.

La détection peut être réalisée dans différentes conditions expérimentales, cliniques et/ou diagnostiques. En particulier, la méthode peut être utilisée pour détecter la prédisposition de certains individus à développer des désordres liés au métabolisme des lipides.

Un objet particulier de l'invention concerne ainsi une méthode de détection de la présence, d'une prédisposition ou d'un risque de développer un désordre lié au métabolisme des lipides chez un sujet, comprenant la détection *in vitro* ou la quantification *in vitro (ou ex vivo),* à partir d'un échantillon prélevé sur le sujet, de lipoparticules comprenant l'Apo B48 et l'Apo B100, à l'aide d'un anticorps tel que défini ci-dessus (incluant des fragments ou des dérivés de ce dernier). Les niveaux élevés d'Apo B48 et/ou d'Apo B100 (par comparaison à une valeur moyenne chez les sujets normaux) sont caractéristiques de ce risque élevé de développer des désordres liés au métabolisme des lipides. Cette méthode est particulièrement adaptée pour la détection de la présence, d'une prédisposition ou d'un risque de développement de l'athérosclérose ou d'une pathologie cardiovasculaire telle que par exemple une maladie coronaire.

Un autre objet de l'invention concerne une méthode de détection ou de suivi de la capture cellulaire des LDL et des lipoprotéines riches en triglycérides chez un sujet, comprenant la détection *in vitro* des quantités d'Apo B48 et/ou d'Apo B100 présentes dans des lipoparticules, à l'aide d'un anticorps tel que défini ci-dessus (incluant des fragments ou des dérivés de ce dernier).

Un autre objet de l'invention concerne une méthode de suivi d'un traitement destiné à corriger des désordres liés au métabolisme des lipides chez un sujet, comprenant la mesure, de préférence différentielle, des quantités d'Apo B48 et/ou d'Apo B100 *in vitro,* dans un échantillon provenant dudit sujet, à l'aide d'un anticorps tel que défini ci-dessus (incluant des fragments ou des dérivés de ce dernier), après l'administration dudit traitement audit sujet. L'efficacité du traitement est corrélée au niveau d'Apo B48 et/ou d'Apo B100 chez le sujet. Ces résultats peuvent être corrélés avec la capacité du traitement à réguler les quantités ou l'activité d'Apo B48 et/ou d'Apo B100 ou encore la capacité à restaurer une activité ou un niveau normal d'Apo B48 et/ou d'Apo B100 chez un sujet. Cette méthode est particulièrement adaptée au suivi d'un traitement de l'athérosclérose ou d'une pathologie cardiovasculaire telle que par exemple une maladie coronaire.

Un autre objet de l'invention concerne une méthode d'évaluation de l'état physiologique d'un sujet, e.g. le niveau du métabolisme des lipides chez un sujet, comprenant la détection des niveaux d'Apo B48 et/ou d'Apo B100 *in vitro* dans un échantillon provenant dudit sujet, à l'aide d'anticorps tels que définis ci-dessus (incluant les fragments et les dérivés de ce dernier).

Les anticorps selon l'invention peuvent également être utilisés pour cribler (sélectionner) des composés ou des régimes alimentaires qui sont susceptibles de moduler la concentration sérique ou plasmatique d'Apo B48 et/ou d'Apo B100. Typiquement, la méthode comprend l'administration à un animal ou à un patient d'un composé ou d'un régime et la récupération d'un échantillon biologique provenant de l'animal ou du patient suivi de la détection de la présence et/ou du dosage des quantités d'Apo B48 et d'Apo B100 dans ledit échantillon à l'aide d'un anticorps tel que défini ci-dessus (incluant des fragments ou dérivés de ce dernier).

Comme indiqué ci-dessus, ces méthodes peuvent être réalisées sur différents échantillons (typiquement du plasma ou du sérum) et par ELISA, RIA, EIA, turbidimétrie, etc., ou de préférence grâce à une méthode d'analyse néphélométrique.

Un autre objet de la présente invention comprend l'utilisation d'un anticorps selon l'invention pour la préparation d'une composition destinée à réguler de façon sélective *in vivo,* chez un sujet, l'activité de l'Apo B100. Par exemple pour inhiber la captation des lipoprotéines contenant de l'Apo B100 (via le LDL-récepteur) par les cellules hépatiques ou périphériques, afin de moduler leur apport lipidique, ou pour inhiber l'assemblage intracellulaire et la sécrétion des lipoprotéines (puisque l'Apo B100 est nécessaire pour cet assemblage), diminuant ainsi la production des lipoprotéines dites athérogènes.

On entend notamment par activité de l'Apo B100 son rôle dans l'assemblage et la sécrétion des lipoprotéines dans le plasma (VLDL) et son rôle dans la captation des lipoprotéines par les cellules périphériques.

L'invention comprend en outre une composition pharmaceutique comprenant un anticorps tel que décrit ci-dessus et éventuellement un excipient ou un véhicule acceptable sur le plan pharmaceutique. L'excipient peut être toute solution, suspension, gel, poudre, etc., compatible avec un usage pharmaceutique. On peut citer des solutés isotoniques, des tampons, solutions salines, etc., éventuellement combinées à des agents stabilisants, tels que des protéines ou autres molécules de haut poids moléculaire, par exemple.

Cette invention concerne également un kit comprenant un peptide ou un anticorps tel que décrit ci-dessus. Le kit peut être utilisé pour détecter ou quantifier l'Apo B48 et/ou l'Apo B100 dans tout échantillon.

D'autres aspects et avantages de l'invention seront décrits dans les exemples qui suivent, qui doivent être considérés comme illustratifs et ne limitent pas l'étendue de l'invention.

### Légende des figures

Figure 1 : Spécificité des anticorps Anti-Apo B100. A : SDS PAGE ; B Immuno Blot. Pistes 1 et 2 : Chylomicrons de sujets HTG. Piste 3 : VLDL de sujets normolipidiques.
Figure 2: Méthode d'analyse néphélométrique de l'Apo B: Courbe de calibration.

### Exemples

### 1. Synthèse d'Apo B100

Les peptides synthétiques spécifiques de l'Apo B100 ont été synthétisés à l'aide d'une méthode réalisée en phase solide (Merrifield 1963) sur un synthétiseur automatique de modèle ABI 431 (Applied Biosystems Inc. Foster city. CA, USA) utilisant une stratégie Boc/Bzl, sur 0,5 mmol de résine PAM-Ala. Chaque acide aminé a été couplé deux fois à l'aide de dicyclohexylcarbodiimide/hydroxybenzotriazole sans être « capé ».

Les produits bruts ont ensuite été purifiés et analysés par HPLC en phase inverse sur une colonne Vydac 18 (Interchim, Montluçon, France) comprenant un gradient linéaire allant de 0 à 100% de tampon B (tampon A : 0,05% TFA dans H₂O et tampon B : 0,05 % TFA, avec 60 % de CH₃CN dans H₂O). Les masses moléculaires ont ensuite été analysées à l'aide d'un spectromètre de masse API (Perkin-Elmer, Foster city, CA, USA) équipé d'un simple quadri-pôle et d'une source d'ions électrospray (nebulizer-assisted electrospray) (Sciex, Toronto, Canada).

L'analyse d'acides aminés a été réalisée à l'aide d'un analyseur d'acides aminés Beckman 6300 (Beckman instruments, Fullerton, CA, USA), après hydrolyse à l'aide d'une solution d'HCl 6N contenant 0,25% de phénol à 110°C pendant 24 heures.

### 2. Immunisations

Un antisérum dirigé contre l'Apo B totale a été préparé selon la technique décrite par Fievet et al (Fievet 1984). Brièvement, des LDL dont la densité est située entre 1,030 et 1,053 (ne contenant donc que de l'Apo B100) ont été préparées par ultracentrifugation, puis injectées à des chèvres afin de produire un immun-sérum anti-Apo B totale reconnaissant toutes les formes de l'Apo B.

L'antisérum spécifique de l'Apo B100 a été préparé également chez des chèvres essentiellement selon le protocole décrit par Vaitukaitis et al. (Vaitukaitis, Robbins et al. 1971). Le peptide (SEQ ID NO : 1 ou SEQ ID NO : 2) qui a servi comme antigène a été émulsifié dans un adjuvant complet de Freud et injecté en sous-cutané à des chèvres, à raison de 0,5 mg de peptide par injection, pour les deux premières injections suivies, à quinze jours d'intervalle, d'injections de rappel utilisant le même adjuvant mais avec seulement 0,25 mg de peptide.

### 3. Isolement d'immunoglobulines (IgG) spécifiques de l'Apo B totale et de l'Apo B100.

Les IgG ont été préparées à l'aide du protocole modifié de Ritchie et al. (Ritchie 1972). Les protéines non-immunoglobulines ont été éliminées du sérum immun et les IgG ont été dialysées et concentrées.

Les anticorps peuvent être conservés entre 2 et 8°C par exemple pour une utilisation dans la semaine, ou sous forme congelée à -20°C, par exemple pour une utilisation prolongée jusqu'à un mois. Les immunoglobulines contiennent de l'azoture de sodium.

### 4. Spécificité des anticorps

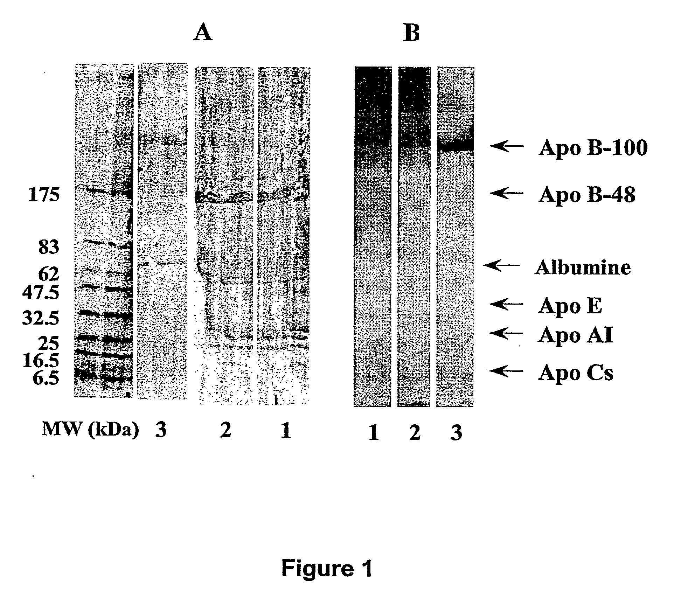

Un immunoblot analytique des chylomicrons et VLDL a été réalisé pour mettre en évidence la spécificité des anticorps anti-Apo B100. Comme le montre la figure 1, aucune réaction croisée n'a pu être observée avec l'Apo B48 ou les autres protéines des chylomicrons. Au niveau des VLDL, l'anticorps anti-Apo B100 ne reconnaît que la protéine située à 550 kDa qui correspond à la masse moléculaire de l'Apo B100. Ces résultats montrent que l'anticorps anti-peptide de SEQ ID NO : 1 reconnaît de façon spécifique l'Apo B100.

### 5. Méthode d'analyse immuno-néphélométrique

### 5.1 Réactifs et matériels utilisés dans le cadre de la méthode d'analyse néphélométrique

**TABLEAU 1**

| | désignation | Références |
|---|---|---|
| Immunoglobulines anti-Apo B | 1 x 6,5 ml (300 tests) | Lot de laboratoire |
| Immunoglobuline anti-Apo B100 | 1 x 5 ml (100 tests) | Lot de laboratoire |
| Standard | 3 x 0,5 ml (900 tests) | Dade-Behring part n° OUPG07 |
| Contrôle | 3 x 0,5 ml (500 tests) | Dade-Behring n° OUPH07 |
| Tampon 1 | 4 x 120 ml (4 x 330 tests) | Beckman Coulter part n° 447650 |
| Cartouche IMMAGE UDR | 10 (10 x 300 tests) | Beckman Coulter part n° 447250 |
| Microtubes | 1000 | Beckman Coulter part n° 448162 |

**Standard :** L'Apo B standard provient d'un sérum humain calibré à l'aide d'une méthode d'électro-immunodiffusion (EIA) et testé pour les virus HIV et hépatites. Ce standard doit être manipulé avec les précautions habituelles en vue d'éviter toute contamination. Le niveau de l'Apo B standard est de 100 mg/dl.
**Préparation :** Pour la construction d'une courbe de calibrage, le standard doit être dilué dans un tampon 1 comme suit.

**TABLEAU 2**

| **Points** | **Concentration (mg/dl)** |
|---|---|
| 1 | 0,156 |
| 2 | 0,312 |
| 3 | 0,6250 |
| 4 | 1,25 |
| 5 | 3,33 |
| 6 | 5 |
| 7 | 10 |
| 8 | 15 |
| 9 | 20 |

**Stockage et stabilité :** L'Apo B standard peut être stockée et conservée à - 20°C. La stabilité est obtenue à l'aide d'EDTA et d'azoture de sodium. La préparation peut être congelée et séchée.
**Préparation des échantillons :** Les échantillons frais sont recommandés pour l'analyse. Les sera doivent être collectés grâce à des procédures établies et utilisées dans les laboratoires cliniques. Si besoin, le sérum peut être stocké entre 2 et 8°C jusqu'à une semaine. Les échantillons conservés congelés peuvent être utilisés pendant une plus longue période ; les échantillons congelés sont stables jusqu'à un an.
**Préparation des échantillons sans particules Apo B100 :** Dans un tube test, on ajoute dans l'ordre suivant : 40 µl d'anticorps anti-Apo B100, 40 µl de sérum et 40 µl de tampon 1. On agite et on incube ensuite la préparation pendant dix minutes à température ambiante, puis on pratique une centrifugation à 3500 tours par minute pendant dix minutes et ensuite on prélève le surnageant qui sera analysé. La concentration finale en Apo B48 dans les échantillons débarrassés des lipoparticules contenant l'Apo B100 est corrigée en fonction de la dilution du surnageant.

### 5.2 Protocole :

- Programmer un réactif défini avec les paramètres listés dans le manuel décrivant la manipulation du système immunochimique IMMAGE,
- Transférer le réactif contenant les anticorps dans le compartiment A de la cartouche,
- Entrer la valeur du standard (la valeur standard actuel d'Apo B est de 1 mg/dl) dans une table de paramétrage, en fonction du schéma de dilution présenté dans le tableau 2,
- Utiliser le tampon 1 comme diluant d'échantillon.

### 5.3 Résumé :

| | |
|---|---|
| **Nom chimique** :Apo B | Unité : |
| | mg/dl |
| Numéro du Lot : | Protocole : |
| Se référer à la cartouche | Néphélométrie non-compétitive |
| Reaget Serial : | Expiration du réactif : |
| Se référer à la cartouche | A définir par l'utilisateur |
| Echantillon ou volume de dilution : | Gain: |
| 20 µl | 3 |
| Tampon réactif: | Dilution: |
| 200 µl | 1/1 |
| Volume du compartiment réactif: | Dilution de l'échantillon : |
| 20µl | 1/15* |
| | 1/1 ** |
| Volume du compartiment B : | Durée de la réaction : |
| 0 µl | 2 minutes |

| | |
|---|---|
| * à configurer après calibrage. ** à configurer après la mesure d'Apo B48. | |

### 5.4 Résultats

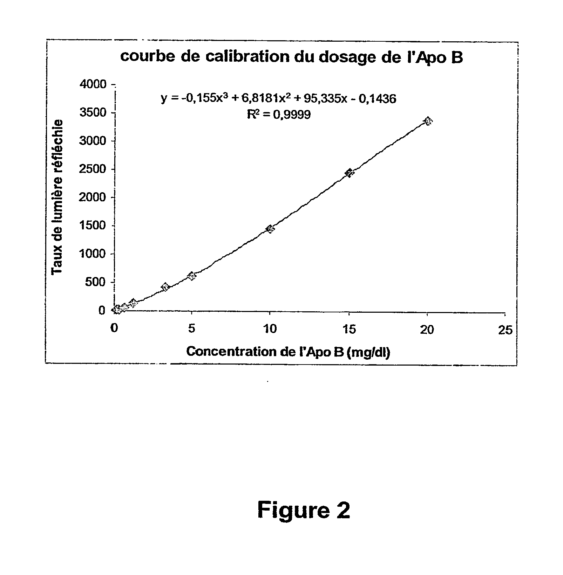

**Courbe de calibrage (Figure 2) :** la courbe de calibrage est élaborée de façon automatique par l'analyseur.
**Contrôle qualité :** Il est recommandé d'utiliser un sérum de contrôle (tel que le sérum de Dade-Behring GmbH, Marburg, Allemagne; destiné au contrôle des apolipoprotéines) pour chaque échantillon testé.
**Valeurs :** l'analyseur indique directement la concentration finale. Pour les échantillons traités contenant de l'anticorps anti-Apo B100, la dilution au tiers doit être corrigée.
**Intervalle de travail :**
   pour l'Apo B totale : 25 à 300 mg/dl
   pour la mesure de l'Apo B48 : 1,5 à 25 mg/dl
   pour la mesure de l'Apo B100 : différence entre les mesures d'Apo B totale et d'Apo B48.
**Coefficient de variation :** 4%
**Valeurs usuelles** (Smith 1997):
   Apo B48 : 4-6 mg/dl pour un sujet normal en état de jeûne
   20 mg/dl pour un sujet se trouvant en période post-prandiale.

### 6 Autres avantages de l'invention.

D'autres avantages et utilisations des peptides synthétiques spécifiques de l'Apo B100 de l'invention incluent :
- la production d'anticorps monoclonaux,
- l'utilisation, comme standard, pour le calibrage de toutes les méthodes d'analyse Apo B100 (ELISA, RIA, électroimmunodiffusion, etc.),
- l'utilisation dans l'investigation des différentes voies métaboliques des lipoprotéines, comme l'enregistrement ou l'inhibition de la capture des lipoprotéines contenant de l'Apo B par les récepteurs LDL.

D'autres avantages et utilisations des anticorps de cette invention sont les suivants :
- l'utilisation dans toutes les méthodes d'analyse immunologiques pour quantifier de façon différentielle l'Apo B100 et l'Apo B48,
- l'utilisation dans le cadre de la détection différentielle de l'Apo B100 et l'Apo B48 (immunoblot, dot blot, immunohistochimie et immunocytochimie),
- l'utilisation dans les méthodes d'immuno-affinité et d'immuno-précipitation pour purifier les protéines.

### Références

Bassiri, R. M., Dvorak J. and Utiger R.D. (1979). Thyrotrpin-releasing hormone. Methods of hormone radioimmunoassay. B. M. In: Jaffe, and Behrman H.R. (Eds). New York, New York Academic Press: p: 46.
Davidson, N. O., S. Anant, et al. (1995). "Apolipoprotein B messenger RNA editing: insights into the molecular regulation of post-transcriptional cytidine deamination." Curr Opin Lipidol **6**(2): 70-4.
Fievet, C., Koffigan M., Ouvry D., Marcovina S., Moschetto Y., Fruchart J.C. (1984). "Noncompetitive enzyme-linked immunoassay for apolipoprotein B in serum." Clin Chem **30**: 98-100.
Goldstein, J. L. and M. S. Brown (1977). "The low-density lipoprotein pathway and its relation to atherosclerosis." Annu Rev Biochem **46**: 897-930.
Harlow, E. a. L., D., Ed. (1988). Antibodies: a Laboratory Manual. New York, Cold Spring Harbor Laboratory Publications.
Havel, R. J., and Kane,J.P. (1995). Introduction: structure and metabolism of plasma lipoproteins. The metabolic and Molecular Bases of inherited Disease. A. L. B. C.R.Scriver, W.S.Sly, and D.Valle. New York, McGraw-Hill. **2**: 1841-1851.
Hidaka, H., H. Kojima, et al. (1990). "Apolipoprotein B-48 analysis by high-performance liquid chromatography in VLDL: a sensitive and rapid method." Clin Chim Acta **189**(3): 287-96.
Hokanson, J. E. and M. A. Austin (1996). "Plasma triglyceride level is a risk factor for cardiovascular disease independent of high-density lipoprotein cholesterol level: a meta- analysis of population-based prospective studies." J Cardiovasc Risk **3**(2): 213-9.
Jones, P. T., Dear P. H., Foote J., Neuberger M. S. and Winter G. (1986). "Replacing the complementarity-determining regions in a human antibody with those from a mouse." Nature **321**: 522-525.
Kane, J. P. (1983). "Apolipoprotein B: structural and metabolic heterogeneity." Annu Rev Physiol **45**: 637-50.
Karpe, F. and A. Hamsten (1994). "Determination of apolipoproteins B-48 and B-100 in triglyceride-rich lipoproteins by analytical SDS-PAGE." J Lipid Res **35**(7): 1311-7.
Kohler, G. and C. Milstein (1975). "Continuous cultures of fused cells secreting antibody of predefined specificity." Nature **256**(5517): 495-7.
Levinson, S. S. and S. G. Wagner (1993). "Immunonephelometric/turbidimetric apolipoprotein B assays for the clinical laboratory." Clin Chim Acta **223**(1-2): 31-42.
Li, B., Z. Wu, et al. (1997). "[Quantitation of human apolipoprotein B100 by immunoturbidimetric assay]." Hua Xi Yi Ke Da Xue Xue Bao **28**(1): 109-12.
Merrifield, R. B. (1963). "Solide phase peptide synthesis. The synthesis of a tetrapeptide." J. Am. Chem. Soc **85**: 2149-2154.
Proctor, S. D. and J. C. Mamo (1997). "Separation and quantification of apolipoprotein B-48 and other apolipoproteins by dynamic sieving capillary electrophoresis." J Lipid Res **38**(2): 410-4.
Riechmann, L., Clark M., Waldmann H. and Winter G. (1988). "Monoclonal antibody therapeutic trials in seven patients with T-cell lymphoma." Nature **332**: 323-327.
Ritchie, R. F., and J. Stevens (1972). Qualifications for acceptable anti-serum performence in the automated immunprecipitatin system: A brief review of commercially available reagents. in advances in Automated Analysis, Technicon Symposia. Tary-town, NY, Mediad Inc: 9-14.
Simons, L. A., T. Dwyer, et al. (1987). "Chylomicrons and chylomicron remnants in coronary artery disease: a case-control study." Atherosclerosis **65**(1-2): 181-9.
Smith, D., Proctor S.D., Mamo J.C. (1997). "A highly sensitive assay for quantitation of apolipoprotein B48 using an antibody to human apolipoprotein B and enhanced chemiluminescence." Ann Clin Biochem. **34**: 185-189.
Smith, D., S. D. Proctor, et al. (1997). "A highly sensitive assay for quantitation of apolipoprotein B48 using an antibody to human apolipoprotein B and enhanced chemiluminescence." Ann Clin Biochem **34**(Pt 2): 185-9.
Uchida, Y., Y. Kurano, et al. (1998). "Establishment of monoclonal antibody against human Apo B-48 and measurement of Apo B-48 in serum by ELISA method." J Clin Lab Anal **12**(5): 289-92.
Vaitukaitis, J., J. B. Robbins, et al. (1971). "A method for producing specific antisera with small doses of immunogen." J Clin Endocrinol Metab **33**(6): 988-91.
Wagner, H. A., R. Nustede, et al. (1987). "Isolation of apolipoprotein-B-48 from chylomicrons by high-performance liquid chromatography." J Chromatogr **397**: 405-8.
Ward, E. S., D. Gussow, et al. (1989). "Binding activities of a repertoire of single immunoglobulin variable domains secreted from Escherichia coli." Nature **341**(6242): 544-6.
Young, S. G. (1990). "Recent progress in understanding apolipoprotein B." Circulation **82**(5): 1574-94.

## Revendications

1. Peptide synthétique substantiellement pur, **caractérisé en ce qu'**il comprend la séquence d'acides aminés SEQ ID NO : 1 ou SEQ ID NO : 2 et comprend moins de 200 acides aminés.

2. Peptide synthétique substantiellement pur, **caractérisé en ce qu'**il consiste en la séquence d'acides aminés SEQ ID NO : 1 ou SEQ ID NO : 2.

3. Peptide selon la revendication 1 ou 2, **caractérisé en ce que** le peptide est soluble ou complexé à une molécule porteuse telle que KLH, la sérum-albumine ou une bille.

4. Peptide selon la revendication 1 à 3, **caractérisé en ce qu'**il permet de générer des anticorps immuno-précipitant l'ApoB100.

5. Anticorps spécifique d'un peptide selon l'une des revendications 1 à 4, ou fragment ou dérivé dudit anticorps présentant substantiellement la même spécificité antigénique.

6. Anticorps selon la revendication 5, **caractérisé en ce qu'**il est produit par immunisation d'un animal non-humain avec un peptide ou un mélange de peptides selon l'une des revendications 1 à 4, et récupération des anticorps ou des cellules productrices d'anticorps.

7. Anticorps selon l'une des revendications 5 ou 6, **caractérisé en ce qu'**il s'agit d'un anticorps polyclonal.

8. Anticorps selon l'une des revendications 5 ou 6, **caractérisés en ce qu'**ils correspondent à un mélange d'anticorps monoclonaux

9. Anticorps selon l'une des revendications 5 à 8, **caractérisé en ce qu'**il présente la capacité de précipiter l'Apo B100.

10. Méthode de production d'un anticorps anti-Apo B100 spécifique, comprenant l'administration d'un peptide selon l'une des revendications 1 à 4 à un animal non-humain et la récupération des anticorps ou des cellules productrices d'anticorps anti-Apo B100 spécifique.

11. Méthode de production d'un anticorps anti-Apo B100 spécifique, comprenant l'administration d'un mélange de peptides selon l'une des revendications 1 à 4 à un animal non-humain et la récupération des anticorps ou des cellules productrices d'anticorps anti-Apo B100 spécifique.

12. Méthode de détection ou de quantification de l'Apo B100 dans un échantillon biologique, comprenant la mise en contact de l'échantillon avec un anticorps selon l'une des revendications 5 à 9 ou avec un anticorps produit grâce à une méthode selon l'une des revendications 10 ou 11, et la détection de la présence de complexes immuns antigène-anticorps.

13. Méthode de détection ou de quantification différentielle de l'Apo B100 et de l'Apo B48 dans un échantillon, comprenant les étapes suivantes :
(a) détermination de la quantité d'Apo B totale dans un échantillon avec un anticorps anti-Apo B totale,
(b) élimination de l'Apo B100 ou des lipoparticules contenant l'Apo B100 dudit échantillon, à l'aide d'un anticorps anti-Apo B100 spécifique selon l'une des revendications 5 à 9 ou produit grâce à une méthode selon l'une des revendications 10 ou 11, et
(c) détermination de la quantité d'Apo B48 dans l'échantillon obtenu à l'issue de l'étape (b) ou dans les lipoparticules ne contenant pas d'Apo B100 au sein de cet échantillon, avec un anticorps anti-Apo B totale.

14. Méthode selon la revendication 13, **caractérisée en ce que**, au cours des étapes a) et/ou c), on utilise un anticorps anti-Apo B totale polyclonal.

15. Méthode selon la revendication 13 ou 14, **caractérisée en ce que** l'anticorps anti-Apo B100 spécifique utilisé au cours de l'étape b) est un anticorps polyclonal ou un mélange d'anticorps monoclonaux ou un anticorps modifié permettant la précipitation des complexes immuns.

16. Méthode selon la revendication 15, **caractérisée en ce que** l'anticorps anti-Apo B100 spécifique utilisé au cours de l'étape b) est un anticorps polyclonal immuno-précipitant.

17. Méthode selon l'une des revendications 12 à 16, **caractérisée en ce que** la présence d'un complexe immun antigène-anticorps est déterminée par ELISA, RIA, EIA, turbidimétrie ou tout autre dosage immunologique.

18. Méthode selon l'une des revendications 12 à 16, **caractérisée en ce que** la présence d'un complexe immun antigène-anticorps est déterminée par une méthode d'analyse néphélométrique.

19. Méthode de détection ou de quantification différentielle de l'Apo B100 et de l'Apo B48 dans un échantillon biologique comprenant la mise en contact dudit échantillon avec un anticorps selon l'une des revendications 5 à 9 ou avec un anticorps produit par une méthode selon l'une des revendications 10 ou 11, et la détection indirecte de la formation de complexes immuns Apo B100-anticorps par une méthode d'analyse néphélométrique.

20. Méthode selon l'une des revendications 12 à 19, **caractérisée en ce que** l'échantillon biologique est un échantillon de sang ou de plasma ou de sérum ou de fluide interstitiel ou de liquide céphalorachidien ou un surnageant de culture cellulaire.

21. Méthode de détection de la présence, d'une prédisposition ou d'un risque de développer un désordre lié au métabolisme des lipides chez un sujet, comprenant la quantification différentielle *in vitro* des quantités d'Apo B48 et d'Apo B100 dans un échantillon prélevé sur un sujet, avec un anticorps selon l'une des revendications 5 à 9, ou avec un anticorps produit par une méthode selon l'une des revendications 10 ou 11.

22. Méthode selon la revendication 21, pour la détection de la présence, d'une prédisposition ou d'un risque de développement de l'athérosclérose ou d'une pathologie cardiovasculaire telle que par exemple une maladie coronaire.

23. Méthode de suivi d'un traitement destiné à corriger des désordres liés au métabolisme des lipides chez un sujet, comprenant la quantification de l'Apo B100 et/ou de l'Apo B48 *in vitro,* dans un échantillon provenant dudit sujet, à l'aide d'un anticorps selon l'une des revendications 5 à 9 ou d'un anticorps produit par une méthode selon l'une des revendications 10 ou 11.

24. Méthode selon la revendication 23, pour le suivi d'un traitement de l'athérosclérose ou d'une pathologie cardiovasculaire telle que par exemple une maladie coronaire.

25. Méthode de détection ou de suivi de la capture cellulaire des LDL et des lipoprotéines riches en triglycérides chez un sujet, comprenant la détection *in vitro* des quantités d'Apo B48 et/ou d'Apo B100 présentes dans des lipoparticules, à l'aide d'un anticorps selon l'une des revendications 5 à 9 ou d'un anticorps produit par une méthode selon l'une des revendications 10 ou 11.

26. Méthode d'évaluation de l'état physiologique d'un sujet, comprenant la détection des niveaux d'Apo B48 et/ou d'Apo B100 *in vitro* dans un échantillon provenant dudit sujet, à l'aide d'anticorps selon l'une des revendications 5 à 9 ou d'un anticorps produit par une méthode selon l'une des revendications 10 ou 11.

27. Utilisation d'anticorps selon l'une des revendications 5 à 9 ou produits par une méthode selon l'une des revendications 10 ou 11 pour cribler des composés ou des régimes alimentaires capables de moduler la concentration sérique ou plasmatique d'Apo B48 et/ou d'Apo B100.

28. Anticorps selon l'une des revendications 5 à 9 ou produit par une méthode selon l'une des revendications 10 ou 11, **caractérisé en ce que** l'anticorps est couplé à des fragments hétérologues tels que des toxines, des marqueurs, des médicaments ou tout autre agent thérapeutique.

29. Utilisation d'un anticorps selon l'une des revendications 5 à 9 et 28 ou produit par une méthode selon l'une des revendications 10 ou 11, pour la préparation d'une composition destinée à réguler de façon sélective *in vivo,* chez un sujet, l'activité de l'Apo B100.

30. Composition pharmaceutique comprenant un anticorps selon l'une des revendications 5 à 9 et 28 ou produit par une méthode selon l'une des revendications 10 ou 11 et un excipient ou un véhicule acceptable sur le plan pharmaceutique.

31. Utilisation d'un peptide selon l'une des revendications 1 à 4 dans le cadre de méthodes de criblage de composés modulant l'activité de l'Apo B.

32. Utilisation d'un peptide selon l'une des revendications 1 à 4 pour la préparation d'une composition destinée à moduler de manière spécifique l'activité de l'Apo B100.

33. Kit comprenant un peptide selon l'une des revendications 1 à 4 ou un anticorps selon l'une des revendications 5 à 9 et 28 ou produit par une méthode selon l'une des revendications 10 ou 11.

## Claims

1. A substantially pure synthetic peptide, **characterized in that** it comprises the amino acid sequence SEQ ID NO : 1 or SEQ ID NO : 2 and comprises less than 200 amino acids.

2. A substantially pure synthetic peptide, **characterized in that** it is consisting of the amino acid sequence SEQ ID NO : 1 or SEQ ID NO : 2.

3. The peptide according to claim 1 or 2, **characterized in that** the peptide is soluble or complexed with a carrier molecule such as KLH, serum albumin or a bead.

4. The peptide according to any one of claims 1 to 3, **characterized in that** it allows the production of Apo-B100 immunoprecipitant antibodies.

5. An antibody specific of a peptide according to any one of claims 1 to 4, or fragment or derivative of said antibody having substantially the same antigenic specificity.

6. The antibody according to claim 5, **characterized in that** it is produced by immunizing a non-human animal with a peptide or a mixture of peptides according to any one of claims 1 to 4, and recovering the antibodies or the antibody-producing cells.

7. The antibody according to any one of claims 5 or 6, **characterized in that** it is a polyclonal antibody.

8. The antibody according to any one of claims 5 or 6, **characterized in that** it corresponds to a mixture of monoclonal antibodies.

9. The antibody according to any one of claims 5 to 8, **characterized in that** it is capable of precipitating Apo-B100.

10. A method for producing a specific anti-Apo-B100 antibody, comprising administering a peptide according to any one of claims 1 to 4 to a non-human animal and recovering the specific anti-Apo-B100 antibodies or antibody-producing cells.

11. A method for producing a specific anti-Apo-B100 antibody, comprising administering a mixture of peptides according to any one of claims 1 to 4 to a non-human animal and recovering the specific anti-Apo-B100 antibodies or antibody-producing cells.

12. A method for detecting or quantifying Apo-B100 in a biological sample, comprising contacting the sample with an antibody according to any one of claims 5 to 9 or with an antibody produced by a method according to any one of claims 10 or 11, and detecting the presence of antigen-antibody immune complexes.

13. A method for detecting or differentially quantifying Apo-B100 and Apo-B48 in a sample, comprising the following steps :
(a) determining the amount of total Apo-B in a sample with a total anti-Apo-B antibody,
(b) eliminating Apo-B100 or lipoparticles containing Apo-B100 from said sample, with the help of a specific anti-Apo-B100 antibody according to any one of claims 5 to 9 or produced by a method according to any one of claims 10 or 11, and
(c) determining the amount of Apo-B48 in the sample obtained after step (b) or in lipoparticles not containing Apo-B100 in this sample, with a total anti-Apo-B antibody.

14. The method according to claim 13, **characterized in that**, during steps (a) and/or (c), a polyclonal antibody directed against total anti-Apo-B is used.

15. The method according to any one of claims 13 or 14, **characterized in that** the specific anti-Apo-B100 antibody used in step (b) is a polyclonal antibody or a mixture of monoclonal antibodies or a modified antibody allowing precipitation of immune complexes.

16. The method according to claim 15, **characterized in that** the specific anti-Apo-B100 antibody used in step (b) is an immunoprecipitant polyclonal antibody.

17. The method according to any one of claims 12 to 16, **characterized in that** the presence of an antigen-antibody immune complex is determined by ELISA, RIA, EIA, turbidimetry or any other immunological assay.

18. The method according to any one of claims 12 to 16, **characterized in that** the presence of an antigen-antibody immune complex is determined by a nephelometric analytical method.

19. A method for detecting or differentially quantifying Apo-B100 and Apo-B48 in a biological sample comprising contacting said sample with an antibody according to any one of claims 5 to 9 or with an antibody produced by a method according to any one of claims 10 or 11, and indirectly detecting the formation of Apo-B100-antibody immune complexes by a nephelometric analytical method.

20. The method according to any one of claims 12 to 19, **characterized in that** the biological sample is a sample of blood or plasma or serum or interstitial fluid or cerebrospinal fluid or a cell culture supernatant.

21. A method for detecting the presence, a predisposition or a risk of developing a disorder of lipid metabolism in a subject, comprising differentially quantifying *in vitro* the amounts of Apo-B48 and Apo-B100 in a sample from a subject, with an antibody according to any one of claims 5 to 9, or with an antibody produced by a method according to any one of claims 10 or 11.

22. The method according to claim 21, for detecting the presence, a predisposition or a risk of developing atherosclerosis or a cardiovascular disease such as a coronary disease for example.

23. A method for monitoring a treatment aimed at correcting disorders of lipid metabolism in a subject, comprising quantifying Apo-B100 and/or Apo-B48 *in vitro*, in a sample from said subject, with the help of an antibody according to any one of claims 5 to 9 or an antibody produced by a method according to any one of claims 10 or 11.

24. The method according to claim 23, for monitoring a treatment of atherosclerosis or of a cardiovascular disease such as a coronary disease for example.

25. A method for detecting or monitoring the cellular uptake of LDL and triglyceride-rich lipoproteins in a subject, comprising detecting *in vitro* the amounts of Apo-B48 and/or Apo-B100 present in lipoparticles, with the help of an antibody according to any one of claims 5 to 9 or an antibody produced by a method according to any one of claims 10 or 11.

26. A method for evaluating the physiological state of a subject, comprising detecting the levels of Apo-B48 and Apo-B100 *in vitro* in a sample from said subject, with the help of an antibody according to any one of claims 5 to 9 or an antibody produced by a method according to any one of claims 10 or 11.

27. A use of antibodies according to any one of claims 5 to 9 or produced by a method according to any one of claims 10 or 11 for screening compounds or diets capable of modulating the serum or plasma concentration of Apo-B48 and/or Apo-B100.

28. The antibody according to any one of claims 5 to 9 or produced by a method according to any one of claims 10 or 11, **characterized in that** the antibody is coupled with heterologous fragments such as toxins, labels, medicaments or any other therapeutic agent.

29. A use of an antibody according to any one of claims 5 to 9 and 28 or produced by a method according to any one of claims 10 or 11, for preparing a composition designed to selectively regulate Apo-B100 activity *in vivo,* in a subject.

30. A pharmaceutical composition comprising an antibody according to any one of claims 5 to 9 and 28 or produced by a method according to any one of claims 10 or 11 and a pharmaceutically acceptable vehicle or excipient.

31. A use of a peptide according to any one of claims 1 to 4 in screening methods for compounds modulating Apo-B activity.

32. A use of a peptide according to any one of claims 1 to 4 for preparing a composition designed to specifically modulate Apo-B100 activity.

33. A kit comprising a peptide according to any one of claims 1 to 4 or an antibody according to any one of claims 5 to 9 and 28 or produced by a method according to any one of claims 10 or 11.

## Patentansprüche

1. Im Wesentlichen reines synthetisches Peptid, **dadurch gekennzeichnet, dass** es die Aminosäure-Sequenz SEQ ID Nr. 1 oder SEQ ID Nr. 2 und weniger als 200 Aminosäuren umfasst.

2. Im Wesentlichen reines synthetisches Peptid, **dadurch gekennzeichnet, dass** es aus der Aminosäure-Sequenz SEQ ID Nr. 1 oder SEQ ID Nr. 2 besteht.

3. Peptid nach Anspruch 1 oder 2, **dadurch gekennzeichnet, dass** es löslich ist oder komplex gebunden ist an ein Trägermolekül, wie z.B. KLH, SerumAlbumin oder eine Kugel.

4. Peptid nach den Ansprüchen 1 bis 3, **dadurch gekennzeichnet, dass** es die Bildung von Antikörpern, die Apo B100 immun-präzipitieren (ausfällen), erlaubt.

5. Antikörper der für ein Peptid nach einem der Ansprüche 1 bis 4 spezifisch ist, oder Fragment oder Derivat dieses Antikörpers, der (das) im Wesentlichen die gleiche Antigen-Spezifität aufweist.

6. Antikörper nach Anspruch 5, **dadurch gekennzeichnet, dass** er gebildet worden ist durch Immunisieren eines Nicht-Human-Tieres mit einem Peptid oder einer Mischung von Peptiden nach einem der Ansprüche 1 bis 4 und Gewinnung (Abtrennung) der Antikörper oder der Antikörper bildenden Zellen.

7. Antikörper nach einem der Ansprüche 5 oder 6, **dadurch gekennzeichnet, dass** es sich um einen polyklonalen Antikörper handelt.

8. Antikörper nach einem der Ansprüche 5 oder 6, **dadurch gekennzeichnet, dass** sie einer Mischung von monoklonalen Antikörpern entsprechen.

9. Antikörper nach einem der Ansprüche 5 bis 8, **dadurch gekennzeichnet, dass** er die Fähigkeit hat, Apo B100 zu präzipitieren (auszufällen).

10. Verfahren zur Herstellung eines spezifischen Anti-Apo B100-Antikörpers, das umfasst die Verabreichung eines Peptids nach einem der Ansprüche 1 bis 4 an ein Nicht-Human-Tier und die Gewinnung (Abtrennung) der Antikörper oder der Zellen, die einen spezifischen Anti-Apo B100-Antikörper bilden.

11. Verfahren zur Herstellung eines spezifischen Anti-Apo B100-Antikörpers, das umfasst die Verabreichung einer Mischung von Peptiden nach einem der Ansprüche 1 bis 4 an ein Nicht-Human-Tier und die Gewinnung (Abtrennung) von Antikörpern oder Zellen, die einen spezifischen Anti-Apo B100-Antikörper bilden.

12. Verfahren zum Nachweis oder zur quantitativen Bestimmung von Apo B100 in einer biologischen Probe, das umfasst das Inkontaktbringen der Probe mit einem Antikörper nach einem der Ansprüche 5 bis 9 oder mit einem Antikörper, der nach einem Verfahren nach einem der Ansprüche 10 oder 11 hergestellt worden ist, und den Nachweis der Anwesenheit von Antigen-Antikörper-Immunkomplexen.

13. Verfahren zum differentiellen Nachweis oder zur differentiellen quantitativen Bestimmung von Apo B100 und von Apo B48 in einer Probe, das die folgenden Stufen umfasst:
(a) Bestimmung der Gesamtmenge von Apo B in einer Probe mit einem Anti-Apo B-Gesamt-Antikörper,
(b) Eliminierung von Apo B100 oder von Lipid-Teilchen, die Apo B100 enthalten, aus der Probe mit Hilfe eines spezifischen Anti-Apo B100-Antikörpers nach einem der Ansprüche 5 bis 9 oder eines spezifischen Anti-Apo B100-Antikörpers, der nach einem Verfahren nach einem der Ansprüche 10 oder 11 hergestellt worden ist, und
(c) Bestimmung der Menge von Apo B48 in der Probe, die in der Stufe (b) erhalten worden ist, oder in Lipid-Teilchen, die kein Apo B100 in dieser Probe enthalten, mit einem Anti-Apo B-Gesamt-Antikörper.

14. Verfahren nach Anspruch 13, **dadurch gekennzeichnet, dass** man in den Stufen (a) und/oder (c) einen polyklonalen Anti-Apo B-Gesamt-Antikörper verwendet.

15. Verfahren nach Anspruch 13 oder 14, **dadurch gekennzeichnet, dass** es sich bei dem in der Stufe (b) verwendeten spezifischen Anti-Apo B100-Antikörper um einen polyklonalen Antikörper oder um eine Mischung von monoklonalen Antikörpern oder um einen Antikörper handelt, der so modifiziert worden ist, dass er die Präzipitation von Immunkomplexen erlaubt.

16. Verfahren nach Anspruch 15, **dadurch gekennzeichnet, dass** der spezifische Anti-Apo B100-Antikörper, der in der Stufe (b) verwendet wird, ein polyklonaler immun-präzipitierender Antikörper ist.

17. Verfahren nach einem der Ansprüche 12 bis 16, **dadurch gekennzeichnet, dass** die Anwesenheit eines Antigen-Antikörper-Immunkomplexes durch ELISA, RIA, EIA, Turbidimetrie oder irgendein anderes immunologisches Analyseverfahren bestimmt wird.

18. Verfahren nach einem der Ansprüche 12 bis 16, **dadurch gekennzeichnet, dass** die Anwesenheit eines Antigen-Antikörper-Immunkomplexes bestimmt wird durch ein nephelometrisches Analyseverfahren.

19. Verfahren zum differentiellen Nachweis oder zur differentiellen quantitativen Bestimmung von Apo B100 und von Apo B48 in einer biologischen Probe, das umfasst das Inkontaktbringen der genannten Probe mit einem Antikörper nach einem der Ansprüche 5 bis 9 oder mit einem Antikörper, der nach einem Verfahren nach einem der Ansprüche 10 oder 11 hergestellt worden ist, und den indirekten Nachweis der Bildung von Apo B100-Antikörper-Immunkomplexen unter Anwendung eines nephelometrischen Analyseverfahrens.

20. Verfahren nach einem der Ansprüche 12 bis 19, **dadurch gekennzeichnet, dass** es sich bei der biologischen Probe um eine Blut- oder Plasma- oder Serum-Probe oder um eine Probe aus einer interstitiellen Flüssigkeit oder Cephalorachidin-Flüssigkeit oder einem Überstand einer Zellkultur handelt.

21. Verfahren zum Nachweis des Vorliegens, einer Prädisposition oder eines Risikos der Entwicklung einer Störung, die im Zusammenhang steht mit dem Lipid-Stoffwechsel bei einem Patienten (Individuum), wobei das Verfahren umfasst die differentielle quantitative in vitro-Bestimmung der Mengen von Apo B48 und Apo B100 in einer aus einem Patienten (Individuum) entnommenen Probe mit einem Antikörper nach einem der Ansprüche 5 bis 9 oder mit einem Antikörper, der nach einem Verfahren nach einem der Ansprüche 10 oder 11 hergestellt worden ist.

22. Verfahren nach Anspruch 21 für den Nachweis des Vorliegens, einer Prädisposition oder eines Risikos der Entwicklung von Arteriosklerose oder einer Cardiovasculär-Erkrankung, wie beispielsweise einer Coronar-Erkrankung.

23. Verfahren zum Verfolgen einer Behandlung, die dazu bestimmt ist, Störungen zu korrigieren, die mit dem Lipid-Stoffwechsel bei einem Patienten (Individuum) im Zusammenhang stehen, das umfasst die quantitative in vitro-Bestimmung von Apo B100 und/oder von Apo B48 in einer Probe, die aus dem genannten Patienten (Individuum) stammt, mit Hilfe eines Antikörpers nach einem der Ansprüche 5 bis 9 oder eines Antikörpers, der nach einem Verfahren nach einem der Ansprüche 10 oder 11 hergestellt worden ist.

24. Verfahren nach Anspruch 23 zum Verfolgen einer Behandlung einer Arteriosklerose oder einer Cardiovasculär-Erkrankung, wie z.B. einer Coronar-Erkrankung.

25. Verfahren zum Nachweis oder zum Verfolgen des zellulären Einfangens von LDL und Lipoproteinen, die reich an Triglyceriden sind, bei einem Patienten (Individuum), das umfasst den in vitro-Nachweis der Gehalte von Apo B48 und/oder Apo B100, die in Lipid-Teilchen enthalten sind, mit Hilfe eines Antikörpers nach einem der Ansprüche 5 bis 9 oder eines Antikörpers, der nach einem Verfahren nach einem der Ansprüche 10 oder 11 hergestellt worden ist.

26. Verfahren zur Bewertung des physiologischen Zustandes eines Patienten (Individuum s), das umfasst den in vitro-Nachweis der Gehalte von Apo B48 und/oder Apo B100 in einer Probe, die aus dem genannten Patienten (Individuum) stammt, mit Hilfe von Antikörpern nach einem der Ansprüche 5 bis 9 oder eines Antikörpers, der nach einem Verfahren nach einem der Ansprüche 10 oder 11 hergestellt worden ist.

27. Verwendung von Antikörpern nach einem der Ansprüche 5 bis 9 oder von Antikörpern, die nach einem Verfahren nach einem der Ansprüche 10 oder 11 hergestellt worden sind, zum Selektionieren (Klassieren) von Verbindungen oder Lebensmitteldiäten, welche die Serum- oder Plasma-Konzentration von Apo B48 und/oder von Apo B100 modulieren können.

28. Antikörper nach einem der Ansprüche 5 bis 9 oder Antikörper, der nach einem Verfahren nach einem der Ansprüche 10 oder 11 hergestellt worden ist, **dadurch gekennzeichnet, dass** der Antikörper an heterologe Fragmente, wie z.B. Toxine, Markierungsstoffe, Arzneimittel oder irgendein anderes therapeutisches Agens, gekoppelt ist.

29. Verwendung eines Antikörpers nach einem der Ansprüche 5 bis 9 und 28 oder eines Antikörpers, der nach einem Verfahren nach einem der Ansprüche 10 oder 11 hergestellt worden ist, für die Herstellung einer Zusammensetzung, die bestimmt ist für die selektive in vivo-Regulierung (-Steuerung) der Aktivität von Apo B100 bei einem Patienten (Individuum).

30. Pharmazeutische Zusammensetzung, die einen Antikörper nach einem der Ansprüche 5 bis 9 und 28 oder einen Antikörper, der nach einem Verfahren nach einem der Ansprüche 10 oder 11 hergestellt worden ist, und einen Exzipienten oder ein Vehiculum, der (das) pharmazeutisch akzeptabel ist, umfasst.

31. Verwendung eines Peptids nach einem der Ansprüche 1 bis 4 in Verfahren zum Selektionieren (Klassieren) von Verbindungen, welche die Aktivität von Apo B modulieren.

32. Verwendung eines Peptids nach einem der Ansprüche 1 bis 4 zur Herstellung einer Zusammensetzung, die dazu bestimmt ist, die Aktivität von Apo B100 auf spezifische Weise zu modulieren.

33. Kit, das umfasst ein Peptid nach einem der Ansprüche 1 bis 4 oder einen Antikörper nach einem der Ansprüche 5 bis 9 und 28 oder einen Antikörper, der nach einem Verfahren nach einem der Ansprüche 10 oder 11 hergestellt worden ist.
